(19) Europäisches Patentamt
European Patent Office
Office européen des brevets

(11) Veröffentlichungsnummer: **0 266 687 B1**

# (12) EUROPÄISCHE PATENTSCHRIFT

(45) Veröffentlichungstag der Patentschrift: **24.07.91**

(51) Int. Cl.$^5$: **C07C 49/395**, C07C 45/54

(21) Anmeldenummer: **87115952.1**

(22) Anmeldetag: **30.10.87**

## (54) Verfahren zur Herstellung von Cyclopentanon.

(30) Priorität: **07.11.86 DE 3638005**

(43) Veröffentlichungstag der Anmeldung:
**11.05.88 Patentblatt 88/19**

(45) Bekanntmachung des Hinweises auf die Patenterteilung:
**24.07.91 Patentblatt 91/30**

(84) Benannte Vertragsstaaten:
**BE DE FR GB IT NL**

(56) Entgegenhaltungen:
**EP-A- 0 093 853**
**DE-C- 256 622**
**US-A- 2 863 923**
**DE-B- 1 768 138**
**FR-A- 1 270 909**
**US-A- 3 953 514**

**PATENT ABSTRACTS OF JAPAN, Band 4, Nr. 12(C-71), 29. Januar 1980; & JP-A-54 148 740**

(73) Patentinhaber: **BASF Aktiengesellschaft**
**Carl-Bosch-Strasse 38**
**W-6700 Ludwigshafen(DE)**

(72) Erfinder: **Lermer, Helmut, Dr.**
**D 3,4**
**W-6800 Mannheim 1(DE)**
Erfinder: **Hoelderich, Wolfgang, Dr.**
**Mannheimer Strasse 18 c**
**W-6710 Frankenthal(DE)**
Erfinder: **Schwarzmann, Matthias, Dr.**
**Carl-Bosch-Strasse 54**
**W-6703 Limburgerhof(DE)**

EP 0 266 687 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Cyclopentanon durch Umsetzung von Adipinsäureestern an zeolithischen Katalysatoren oder an Phosphat-Katalysatoren.

Es ist bekannt, Cyclopentanon durch Erhitzen von Adipinsäure in Gegenwart von katalytischen Mengen Schwermetallsalzen in der Flüssigphase herzustellen. Als Metalle werden hierbei Barium oder Thorium verwendet. Ein Nachteil für die Umsetzung dieses Verfahrens in die Technik sind die bei den hohen Temperaturen auftretenden Korrosionsprobleme und die Verwendung von giftigen Schwermetallen.

Es wurde nun gefunden, daß man Cyclopentanon in vorteilhafter Weise herstellen kann, wenn man Adipinsäureester der Formel

$R^1OOC—(CH_2)_4—COOR^2$ (I),

in der $R^1$ und $R^2$ Alkylreste mit 1 bis 12 Kohlenstoffatomen, Cycloalkylreste mit 5 oder 6 Kohlenstoffatomen, Aralkyl- oder Arylreste und $R^2$ zusätzlich auch Wasserstoff bedeuten, bei Temperaturen von 150 °C bis 450 °C in Gegenwart von zeolithischen Katalysatoren und oder Phosphat-Katalysatoren umsetzt.

Die erfindungsgemäße Reaktion kann man beispielsweise für die Umsetzung von Adipinsäuredimethylester zu Cyclopentanon durch folgende Reaktionsgleichung darstellen:

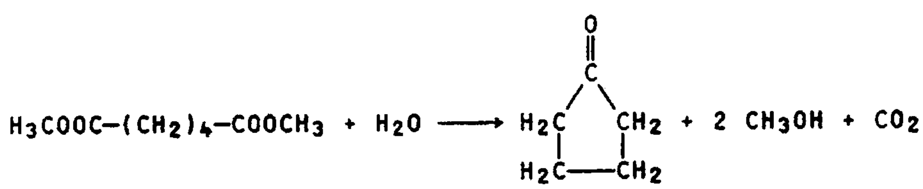

Bisher war lediglich bekannt, Adipinsäureester mit Hilfe stöchiometrischer Mengen starker Basen wie Natriumalkoholaten oder Natriumamiden über das Reaktionszwischenprodukt Cyclopentanon-2-carbonsäuremethylester in Cyclopentanon zu überführen (Dieckmann-Kondensation). Hierbei sind drei Verfahrensschritte notwendig. Darüber hinaus muß die verwendete Base neutralisiert werden, so daß ein erheblicher Zwansanfall an Neutralsalzen auftritt.

Die glatte katalytische Umsetzung von Adipinsäureestern zu Cyclopentanon ist überraschend. Es war nicht vorherzusehen, daß diese Reaktion an zeolithischen Katalysatoren oder Phosphat-Katalysatoren einstufig und mit hoher Ausbeute ablaufen würde.

Erfindungsgemäß kann aliphatische, cycloaliphatische, araliphatische und aromatische Mono- und Diester der Adipinsäure in Cyclopentanon überführen. Beispielhaft seien folgende Reste $R^1$ und $R^2$ in (I) gennant: Methyl-, Ethyl-, Propyl-, Isopropyl-, Butyl-, Isobutyl-, tert.-Butyl-, Hexyl-, Nonyl-, Dodecyl-, Cyclopentyl-, Cyclohexyl-, Phenyl- oder Benzylreste. Danach können folgende Adipinsäureester (I) als Ausgangsstoffe verwendet werden: Adipinsäuredimethylester, Adipinsäuremonomethylester, Adipinsäurediethylester, Adipinsäuredibutylester, Adipinsäuredicyclohexylester oder Adipinsäuredibenzylester.

Als Katalysatoren im Sinne der Erfindung sind im allgemeinen geeignet Zeolithe des Pentasiltyps wie Aluminiumsilikatzeolithe, Borosilikatzeolithe, Einsensilikatzeolithe, und Zeolithe des Faujasit-Typs.

Die Zeolithe können z.B. mit Alkalimetallen, Übergangsmetallen und mit seltenen Erdmetallen dotiert werden.

Man kann als Katalysatoren aber auch Phosphate der Elemente B, Al, Zr, Fe, Sr oder deren Gemische verwenden. Auch hydrothermal hergestellte Phosphate sind z.B. als Katalysatoren geeignet z.B. hydrothermal hergestellte Aluminiumphosphate, Siliciumaluminiumphosphate oder Siliciumeisenaluminiumphosphate. Daneben kann man als Katalysatoren Phosphorsäure auf Trägermaterialien verwenden.

Als Katalysatoren für das erfindungsgemäße Verfahren verwendet man die Zeolithe zweckmäßig in der aciden Form. Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si-und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen oder Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein

vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man Zeolithe vom Type A, L, X oder Y.

Für das erfindungsgemäße Verfahren in Betracht kommende Katalysatoren sind Zeolithe aus der Mordenit-Gruppe oder engporige Zeolithe vom Erionit-bzw. Chabasit-Typ oder Zeolithe vom Faujasit-Typ, z.B. Y-, X- oder L-Zeolithe.

In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40 000. Diese Aluminosilikatzeolithe des Pentasiltyps können auch in etherischem Medium wie Diethylenglykoldimethylether, in alkoholischem Medium wie Methanol bzw. 1,4-Butandiol oder in Wasser synthetisiert werden.

Zu den erfindungsgemäß verwendbaren siliciumreichen Zeolithen ($SiO_2/Al_2O_3 \geqq 10$) gehören auch die verschiedenen ZSM-Typen, Ferrierit, Nu-1 und Silicalit ®.

Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino-bzw., Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameinsensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustrausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man den verformten Zeolithen in einem Steigrohr vorlegt und bei 20 bis 100 °C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wird der getränkte Zeolith bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen, z.B. Pd-, Cu-, Ni-dotierten Zeolithen ist eine Nachbehandlung mit Wasserstoff vorteilhaft.

Eine weiter Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80 °C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische Material vor seiner Vorformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials, wird dieses zweckmäßig, z.B. bei Temperaturen von 100 bis 160 °C, getrocknet und bei Temperaturen von im allgemeinen 450 °C bis 600 °C calciniert. Gemäß einer weiteren bevorzugten Ausführungsform für die Säurebehandlung, wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0,5 bis 5 h, vorzugsweise mit 12 bis 20 Gew.-%iger Salzsäure, behandelt. Zweckmäßig wird das zeolithische Material anschließend ausgewaschen, bei Temperaturen von 100 bis 160 °C getrocknet und bei Temperaturen von 450 bis 600 °C

calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110° C getrocknet und bei 500° C calciniert.

Weitere Katalysatoren für die Herstellung von Cyclopentanon aus Adipinsäureestern sind Phosphate, insbesondere Aluminiumphosphate, Siliciumaluminiumphosphate, Eisenaluminiumphosphate, Cerphosphat, Zirkonphosphate, Borphosphat, Eisenphosphat oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt. Geeignete Aluminiumphosphate sind z.B. APO-5, APO-9, APO-11, APO-12, APO-14, APO-21, APO-25, APO-31 und APO-33.

Beispielsweise $AlPO_4$-5 (APO-5) wird synthetisiert, indem man Orthophosphorsäure mit Pseudoboehmit (Catapal SB ®) in Wasser homogen mischt, zu dieser Mischung Tetrapropylammoniumhydroxid gibt und danach bei etwa 150° C 20 bis 60 h unter autogenem Druck in einem Autoklaven umsetzt. Das abfiltrierte $AlPO_4$ wird getrocknet bei 100 bis 160° C und calciniert bei 450 bis 550° C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei ca. 200° C unter autogenem Druck während 200 bis 400 h synthetisiert. Verwendet man anstelle von DABCO-Lösung Ethylendiamin, so gelangt man zu APO-12.

Die Synthese des $AlPO_4$-21 (APO-21) erfolgt aus Orthophosphorsäure und Pseudoboehmit in wäßriger Pyrrolidin-Lösung bei 150 bis 200° C unter autogenem Druck während 50 bis 200 h.

Für das erfindungsgemäße Verfahren kann man auch bekannte Siliciumaluminiumphosphate wie SAPO-5, SAPO-11, SAPO-31 und SAPO-34 einsetzen. Diese Verbindungen werden hergestellt durch Kristallisation aus wäßriger Mischung bei 100 bis 250° C und autogenem Druck während 2 h bis 2 Wochen, wobei die Reaktionsmischung aus einer Silicium-, Aluminium-und Phosphorkomponente in wäßrigen aminhaltigen Lösungen umgesetzt wird.

SAPO-5 beispielsweise wird durch Mischen von $SiO_2$, suspendiert in wäßriger Tetrapropylammoniumhydroxid-Lösung, mit einer wäßrigen Suspension aus Pseudoboehmit und Orthophosphorsäure und anschließende Umsetzung bei 150 bis 200° C während 20 bis 200 h unter autogenem Druck in einem Rührautoklaven erhalten. Die Trocknung des abfiltrierten Pulvers erfolgt bei 110 bis 160° C und die Calcination bei 450 bis 550° C.

Als Siliciumaluminiumphosphate sind auch ZYT-5, ZYT-6, ZYT-7, ZYT-9, ZYT-11 und ZYT-12 geeignet.

Als Phosphat-Katalysatoren kann man bei dem Verfahren gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3 \times 9\ H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60°/16 h getrocknet.

Borphosphate als Katalysatoren für das erfindungsgemäße Verfahren kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650° C vorzugsweise 300 bis 500° C herstellen.

$CePO_4$ erhält man durch Fällung aus 52 g $Ce(NO_3)_3 \times 6\ H_2O$ und 56 g $NaH_2PO_4 \times 2\ H_2O$. Nach der Filtration wird das material zu Strängen verformt, bei 120° C getrocknet und bei 450° C calciniert. Als Phosphate lassen sich auch $SrHPO_4$, $FePO_4$ und $Zr_3(PO_4)_4$ für das erfindungsgemäße Verfahren verwenden.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Die Reaktion wird vorzugsweise in der Gasphase bei 150 bis 450° C, insbesondere bei 200° C bis 400° C, und einer Belastung WHSV von 0,1 bis 20 $h^{-1}$, vorzugsweise 0,5 bis 5 $h^{-1}$ (g Einsatzgemisch/g Katalysator und Stunde) ausgeführt. Es ist auch möglich, die Reaktion in der Flüssigphase (Suspension-, Riesel- oder Sumpffahrweise) bei Temperaturen zwischen 50 und 200° C durchzuführen.

Das Verfahren wird in der Regel bei Normaldruck oder je nach Flüchtigkeit der Ausgangsverbindung bei vermindertem oder erhöhtem Druck und vorzugsweise kontinuierlich durchgeführt. Schwerflüchtige oder feste Ausgangsstoffe werden in gelöster Form z.B. in THF-, Toluol- oder Petrolether-Lösung zum Einsatz gebracht. Allgemein ist eine Verdünnung des Ausgangsstoffes mit derartigen Lösungsmitteln oder mit Inertgasen wie $N_2$, Ar oder mit $H_2O$-Dampf möglich.

Eine bevorzugte Ausführungsform des erfindungsgemäßen Verfahrens in der Gasphase besteht z.B. darin, daß man ein Gemisch aus Adipinsäureester (I) und Wasser zunächst verdampft und dann, gegebenenfalls zusammen mit einem Inertgas, wie Stickstoff oder Argon, bei der gewünschten Reaktionstemperatur gasförmig in eine in auf- und abwirbelnder Bewegung befindliche Katalysatorschicht (Wirbelschicht) einleitet. Der Reaktionsaustrag wird mittels geeigneter Kühlvorrichtungen kondensiert und anschließend durch fraktionierende Destillation aufgearbeitet. Unumgesetzter Adipinsäureester kann zurückgeführt werden.

Die als Ausgangsstoffe benötigten Adipinsäureester (I) kann man durch Veresterung von Adipinsäure nach bekannten Verfahren oder ausgehend von Butadien durch zweimalige Carbonylierung in Gegenwart von Alkoholen herstellen.

Cyclopentanon stellt ein wertvolles Zwischenprodukt dar. So kann man z.B. durch reduktive Aminierung Cyclopentylamin herstellen, das für die Synthese von Pflanzenschutzmitteln und Pharmazeutika benötigt wird.

Beispiele 1 bis 9

Die Reaktion wird in der Gasphase unter isothermen Bedingungen in einem Rohrreaktor (Wendel, 0,6 cm Innendurchmesser, 90 cm Länge) durchgeführt. Die gasförmigen Reaktionsausträge werden kondensiert, gewogen und gaschromatographisch nach bekannter Methode analysiert.

Die jeweils eingesetzten Katalysatoren sind:

Katalysator A

Ein Aluminiumsilikatzeolith vom Pentasil-Typ wird unter hydrothermalen Bedingungen bei autogenem Druck und 150 °C aus 650 g hochdispersem $SiO_2$, 203 g $Al_2(SO_4)_3 \times 18\ H_2O$ in 10 kg einer wäßrigen 1,6-Diaminohexan-Lösung (Mischung 50:50 Massen-%) in einem Rührautoklaven hergestellt. Nach Abfiltrieren und Auswaschen wird das kristalline Reaktionsprodukt bei 110 °C/24 h getrocknet und bei 500 °C/h calciniert. Dieser Aluminiumsilikatzeolith enthält 92,8 Massen-% $SiO_2$ und 4,2 Massen-% $Al_2O_3$. Mit diesem Material werden durch Verformen mit einem Verformungsmittel 2 mm-Stränge hergestellt, die bei 110 °C/16 h getrocknet und bei 500 °C/24 h calciniert werden.

Katalysator B

Ein Eisensilikatzeolith vom Pentasil-Typ wird unter hydrothermalen Bedingungen bei autogenem Druck und 165 °C aus 273 g Natrium-Wasserglas (27,2 % $SiO_2$, 8,5 % $Na_2O$), 126 g 1,6-Diaminohexan, 551 g Wasser, 20,6 g 96 %iger Schwefelsäure und 31,1 g Eisen(III)-sulfat in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Eisensilikatzeolith besitzt ein $SiO_2/Fe_2O_3$-Verhältnis von 17,7 und einen $Na_2O$-Gehalt von 1,2 Massen-%. Mit diesem Material werden durch Verformen mit pyrogener Kieselsäure (8 Massentiele : 2 Massenteile) und einem Verformungshilfsmittel 2 mm-Stränge hergestellt, die bei 110 °C/16 h getrocknet und bei 500 °C/24 h calciniert werden.

Katalysator C

Ein Borsilikatzeolith des Pentasil-Typs wird in einer hydrothermalen Synthese aus 640 g hochdispersem $SiO_2$, 122 g $H_3BO_3$, 8000 g einer wäßrigen 1,6-Diaminohexan-Lösung (Mischung 50 : 50 Massen-%) bei 170 °C autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borsilikatzeolith setzt sich zusammen aus 94,2 Massen-% $SiO_2$ und 2,3 Massen-% $B_2O_3$.

Mit diesem Material werden durch Verformen mit einem Verformungshilfsmittel 2 mm-Stränge hergestellt, die bei 110 °C/16 h getrocknet und bei 500 °C/24 h calciniert werden.

Die Stränge werden mit einer wäßrigen $Zn(NO_3)_2$-Lösung ausgetauscht, bei 110 °C/16 h getrocknet und bei 500 °C/16 h calciniert. Der Zn-Gehalt beträgt 0,58 Massen-%.

Katalysator D

Der Katalysator D wird wie Katalysator C durch Ionenaustausch der Borsilikatzoelith-Stränge mit einer wäßrigen $Co(NO_3)_2$-Lösung, anschließendes Trocknen bei 110 °C/16 h und Calcinieren bei 500 °C/16 h

hergestellt. Der Co-Gehalt beträgt 0,3 Massen-%.

Katalysator E

Katalysator E wird durch Imprägnieren der borsilikatzeolith-Stränge mit wäßriger $Co(NO_3)_2$-Lösung, anschließendes Trocknen bei 110 °C/16 h und Calcinieren bei 500 °C/16 h hergestellt. Der Co-Gehalt beträgt 0,9 Massen-%.

Katalysator F

Katalysator F ist ein bei pH 8 gefälltes Aluminiumphosphat (42 Massen-% $Al_2O_3$, 58 Massen-% $P_2O_5$). Das Pulver wird unter Zuschlag von 20 % Kaliumpropionat zu 2 mm-Strängen verformt, bei 110 °C/16 h getrocknet und bei 550 °C/1,5 h calciniert.

Katalysator G

Katalysator G wird durch Ionenaustausch der Stränge von Katalysator B mit einer wäßrigen $Co(NO_3)_2$-Lösung, anschließendes Trocknen bei 110 °C/16 h und Calcinieren bei 500 °C/16 h hergestellt. Der Co-Gehalt ist 0,57 Massen-%; der Na-Gehalt ist 0,10 Massen-%.

Tabelle 1 zeigt die mit diesen Katalysatoren erhaltenen Ergebnisse nach jeweils 4 h Versuchszeit.

Tabelle 1: Katalysatortest zur Synthese von Cyclopentanon aus DMA (Adipinsäuredimethylester)

| Beispiele | Katalysator | Temperatur [°C] | WHSV [$h^{-1}$] | Massenverhältnis DMA : THF | Umsatz DMA [%] | Selektivität Cyclopentanon [%] | Ausbeute [%] |
|---|---|---|---|---|---|---|---|
| 1 | A | 400 | 3,7 | 1,0 | 42,6 | 38,5 | 16,4 |
| 2 | B | 350 | 2,7 | 1,0 | 34,1 | 87,8 | 29,9 |
| 3 | B | 400 | 3,2 | 1,0 | 86,5 | 58,2 | 50,4 |
| 4 | B | 450 | 2,4 | 1,0 | 97,8 | 23,7 | 23,2 |
| 5 | C | 400 | 2,6 | 1,0 | 68,3 | 57,8 | 39,5 |
| 6 | D | 400 | 3,1 | - | 29,5 | 43,5 | 12,8 |
| 7 | D | 400 | 2,8 | 1,0 | 41,6 | 73,9 | 30,7 |
| 8 | E | 400 | 3,5 | 1,0 | 56,0 | 59,6 | 33,4 |
| 9 | F | 400 | 2,6 | 1,0 | 75,3 | 57,2 | 43,1 |

Beispiel 10

Bei einer Temperatur von 350 °C werden 46,2 g Adipinsäuredimethylester pro Stunde und eine äquimolare Menge Wasser verdampft und mit einem Stickstoffstrom von 15 l/h über 50 g Katalysator B bei 350 °C geleitet. Der Reaktionsaustrag wird kondensiert, gewogen und gaschromatographisch nach bekannter Methode analysiert.

| Laufzeit | Umsatz DMA | Selektivität Cyclopentanon |
| --- | --- | --- |
| [h] | [%] | [%] |
| 24 | 87,4 | 88,3 |
| 48 | 81,0 | 99,1 |

Beispiel 11

Bei einer Temperatur von 353 °C werden 20,4 g Adipinsäuredimethylester pro Stunde und Wasser im Molverhältnis 1:2 (Adipinsäuredimethylester:Wasser) verdampft und über 50 g Katalysator G bei 353 °C geleitet. Der Reaktionsaustrag wird kondensiert, gewogen und gaschromatographisch nach bekannter Methode analysiert. Die Laufzeit des Katalysators betrug 216 Stunden. Der Umsatz während des Beobachtungszeitraums lag zwischen 99,5 und 100 %, die Selektivität für Cyclopentanon zwischen 75 und 79 %. Während dieser Laufzeit war keine Desaktivierung des Katalysators feststellbar.

## Patentansprüche

1. Verfahren zur Herstellung von Cyclopentanon aus Adipinsäureestern, dadurch gekennzeichnet, daß man Adipinsäureester der Formel

$$R^1OOC—(CH_2)_4—COOR^2 \quad (I),$$

in der $R^1$ und $R^2$ Alkylreste mit 1 bis 12 Kohlenstoffatomen, Cycloalkylreste mit 5 oder 6 Kohlenstoffatomen, Aralkyl- oder Arylreste und $R^2$ zusätzlich Wasserstoff bedeuten kann, in der Gas- oder Flüssigphase bei Temperaturen von 150 °C bis 450 °C, in Gegenwart von zeolithischen Katalysatoren und/oder von Phosphat-Katalysatoren umsetzt.

2. Verfahren nach Anspruch 1, dadurch gekennzeichnet, daß man als Katalysatoren Zeolithe des Pentasil-Typs verwendet.

3. Verfahren nach Anspruch 1, dadurch gekennzeichnet, daß man als Katalysatoren Aluminiumphosphate, Siliciumaluminiumphosphate, Eisenaluminiumphosphate, Borphosphate, Cerphosphate oder Zirkonphosphate verwendet.

4. Verfahren nach Anspruch 3, dadurch gekennzeichnet, daß man als Katalysatoren hydrothermal hergestellte Phosphate mit zeolithähnlicher Struktur verwendet.

## Claims

1. A process for preparing cyclopentanone from an adipic ester, which comprises reacting an adipic ester of the formula

$$R^1OOC\text{-}(CH_2)_4\text{-}COOR^2 \quad (I)$$

where $R^1$ and $R^2$ are each alkyl of 1 to 12 carbon atoms, cycloalkyl of 5 or 6 carbon atoms, aralkyl or aryl and $R^2$ can additionally be hydrogen, in the gas or liquid phase at from 150 °C to 450 °C in the presence of a zeolitic catalyst or a phosphate catalyst.

2. A process as claimed in claim 1, wherein the catalyst used is a zeolite of the pentasil type.

3. A process as claimed in claim 1, wherein the catalyst used is an aluminum phosphate, a silicon aluminum phosphate, an iron aluminum phosphate, a boron phosphate, a cerium phosphate or a zirconium phosphate.

4. A process as claimed in claim 3, wherein the catalyst used is a hydrothermally prepared phosphate having a zeolitelike structure.

## Revendications

1. Procédé de préparation de cyclopentanone à partir d'esters de l'acide adipique, caractérisé en ce que l'on fait réagir des esters de l'acide adipique de formule

$$R^1OOC\text{-}(CH_2)_4\text{-}COOR^2 \quad (I),$$

dans laquelle $R^1$ et $R^2$ désignent des restes alkyle contenant 1 à 12 atomes de carbone, des restes cycloalkyle contenant 5 ou 6 atomes de carbone, des restes aralkyle ou aryle, $R^2$ pouvant complémentairement désigner de l'hydrogène, en phase gazeuse ou liquide, à des températures de 150 °C à 450 °C, en présence de catalyseurs qui sont des zéolites et/ou des phosphates.

2. Procédé suivant la revendication 1, caractérisé en ce que l'on utilise comme catalyseurs des zéolites du type pentasil.

3. Procédé suivant la revendication 1, caractérisé en ce que l'on utilise comme catalyseurs des phosphates d' aluminium, des phosphates de silicium et d'aluminium, des phosphates de fer et d'aluminium, des phosphates de bore, des phosphates de cérium ou des phosphates de zirconium.

4. Procédé suivant la revendication 3, caractérisé en ce que l'on utilise comme catalyseurs des phosphates préparés par voie hydrothermale ayant une structure semblable à celle des zéolites.